# EUROPEAN PATENT APPLICATION

(11) **EP 1 915 992 A1**
(43) Date of publication of application: **30.04.2008**
(21) Application number: 08101136.3
(22) Date of filing: 14.02.2003
(51) Int. Cl.: A61K 31/12, A61K 31/196, A61K 31/352, A61K 31/5415, A61K 31/135, A61P 13/10

(54) **Use of cyclooxygenase inhibitors and antimuscarinic agents for the treatment of incontinence**

(30) Priority: 19.02.2002 US 357888 P
(62) Divisional of application: 03742765.5
(71) Applicant: Pharmacia Corporation, Peapack, NJ 07977 (US)
(72) Inventor: Versi, Ebrahim, Gladstone, NJ 07934 (US)
(74) Representative: Ford, Timothy James

(57) **Abstract**

The present invention provides a method for the use of a cyclooxygenase-2 inhibitor in combination with an anti-muscarinic agent, for the treatment or prophylaxis of interstitial cystitis in a subject in need of such treatment or prevention, comprising administering to the subject an effective amount of the cyclooxygenase-2 inhibitor and the anti-muscarinic agent.

## Description

### BACKGROUND OF THE INVENTION

### Field of the Invention

This invention is in the field of the prevention and treatment of urinary tract disorders. More specifically, this invention relates to the use of cyclooxygenase inhibitors or derivatives thereof in preventing and treating urinary incontinence conditions, in particular, urge incontinence, stress incontinence, mixed incontinence, overactive bladder, neurogenic incontinence, detrusor hyperreflexia, suburethral diverticulitis, and urinary tract infection. This invention also relates to combinations of compounds, compositions, and methods for their use in preventing and treating urinary tract disorders, and more particularly, to the use of anti-muscarinic agents and cyclooxygenase inhibitors in combination with each other for the treatment of urinary incontinence conditions.

### Description of Related Art

Literature and patent references cited in this description of related art, as indicated by numbers in parentheses, are listed sequentially in Table 1 (vide infra) and are herein incorporated by reference.

Although the exact etiology of incontinence conditions related to overactive bladder remains unknown, a primary target for pharmacological therapy has historically been the peripheral nervous system (PNS). It has been known for some time that acetylcholine liberated from cholinergic nerve endings acts via muscarinic receptors as a major mediator of the voiding response in humans. For this reason anticholinergic agents that block muscarinic receptors have found broad utility in the treatment of urinary incontinence, as reviewed by Andersson (1). Anticholinergic therapy has a stabilizing effect on the detrusor muscle of the bladder, decreases the frequency of involuntary detrusor contractions, increases bladder capacity, and does not affect warning time. However, anti-muscarinic agents often lack receptor selectivity and consequently display side effects to varying degrees, including dry mouth, blurred vision, and constipation. A key focus in recent incontinence research has been the development of new anti-muscarinic agents having reduced side effect profiles, as reviewed by Wein (2). An example of such an agent is tolteridine, which has compared favorably in clinical trials with other anti-muscarinic agents as a treatment for incontinence, as discussed by Nilvebrant et al. (3) and Appell (4).

**Table 1. Description of Related Art**

| | **Author (Patent Asignee)** | **Journal (Patent Reference)** |
|---|---|---|
| 1 | | AnderssonExp. Physiol, 84, 195-213 (1999) |
| 2 | | WeinExp. Opin. Invest. Drugs, 10, 65-83 (2001) |
| 3 | | Nilvebrant et al. Eur. J. Pharmacol., 327, 195-207 (1997) |
| 4 | | AppellUrology, 50, 90-96 (1997) |
| 5 | | Palea et al. Br. J. Pharmacol., 124, 865-872 (1998) |
| 6 | | Park et al. Am. J. Physiol., 276, F129-F136 (1999) |
| 7 | | Cardozo and StantonJ. Urol, 123, 399-401 (1980) |
| 8 | | Cardozo et al. Brit. Med. J., 280, 281-282 (1980) |
| 9 | | PalmerJ. Int. Med. Res., 11, 11-17 (1983) |
| 10 | | Somasundaram et al. Gut, 40, 608-613 (1997) |
| 11 | | Mariotto et al. Br. J. Pharmacol., 116, 1713-1714 (1995) |

Extended-release formulations of anti-muscarinic agents have been reported to reduce side effects in the case of both oxybutynin and tolteridine, as discussed by Wein (2).

Another fundamental pathway in the regulation of bladder function involves the biosynthesis of prostaglandins (PGs). PGs are thought to play an important role in endogenous modulation of the micturition reflex. For example, various prostanoids have been shown to contract the bladder detrusor muscle, as discussed by Palea et al. (5). Chronic bladder obstruction has been shown to cause elevated levels of cyclooxygenase-2 in the bladder via increased mechanical stretch, as reported by Park et al. (6).

The non-steroidal anti-inflammatory drugs (NSAIDs) are known to prevent the formation of prostaglandins by inhibiting enzymes in the human arachidonic acid/prostaglandin pathway, in particular the enzyme cyclooxygenase (COX). For this reason the NSAIDs are effective in reducing prostaglandin-mediated neurological responses such as pain sensation and smooth muscle contraction. Preliminary studies of the NSAID COX inhibitor indomethacin have suggested that relief of overactive bladder symptoms can be obtained, although gastrointestinal side effects resulted in discontinuation of the treatment, as discussed by Cardozo and Stanton (7). Similar results were obtained for the NSAID COX inhibitor flurbiprofen by Cardozo et al. (8) and Palmer (9). The effectiveness of the related analog nitroflurbiprofen in controlling overactive bladder in laboratory rats has also been reported, accompanied by a reduced incidence of intestinal ulcers, as disclosed by Somasundaram et al. (10) and Mariotto et al. (11).

The recent discovery that there are two isoforms of the COX enzyme, COX-1 and COX-2, has given rise to new approaches for NSAID discovery and utilization, since it has been shown that COX-2 is the isoform specifically induced in many disease-affected tissues. Various compounds have been identified which have activity as COX-2 inhibitors, and much research continues in this area.

### SUMMARY OF THE INVENTION

While the above references indicate the value of the known therapies in reducing the symptoms of overactive bladder, there is a continuing urgent need to find safe, effective agents for the prophylaxis and treatment of a variety of incontinence-related conditions. The cyclooxygenase inhibitors of the present invention, as well as their novel combinations with anti-muscarinic agents, exhibit improved efficacy, improved potency, and/or reduced dosing requirements for the active compounds relative to incontinence treatment regimens previously disclosed in the published literature.

To address the continuing need to find safe and effective agents for the treatment of urinary incontinence conditions, the use of cyclooxygenase inhibitors in the prophylaxis and treatment of urinary disorders is now provided, as is the use of combination therapies of cyclooxygenase inhibitors and anti-muscarinic drugs.

Among its several embodiments, the present invention provides a method of treating a subject with a urinary incontinence condition effective amount of one or more cyclooxygenase inhibitors or prodrugs thereof.

In a preferred embodiment of the present invention the cyclooxygenase inhibitor is a COX-2 selective cyclooxygenase inhibitor or prodrug thereof.

In another embodiment, the present invention provides a method for the treatment or prophylaxis of a urinary incontinence condition wherein the method comprises treating a patient with an amount of a cyclooxygenase inhibitor and an amount of an anti-muscarinic agent wherein the amount of the cyclooxygenase inhibitor and the amount of the anti-muscarinic agent together constitute a urinary incontinence condition treating or prophylactic-effective amount of the cyclooxygenase inhibitor and the anti-muscarinic agent. In a preferred embodiment the cyclooxygenase inhibitor is a cyclooxygenase-2 selective inhibitor.

In another embodiment, the present invention provides a method for the treatment or prophylaxis of interstitial cystitis in a patient in need of such treatment or prophylaxis, comprising treating the patient with an amount of an anti-muscarinic agent and an amount of a cyclooxygenase inhibitor or prodrug, wherein the amount of the anti-muscarinic agent and the amount of the cyclooxygenase inhibitor together comprise a interstitial cystitis condition treating or prophylactic-effective amount of the anti-muscarinic agent and the cyclooxygenase inhibitor.

In another embodiment, the present invention provides a therapeutic composition comprising an amount of an anti-muscarinic agent and an amount of a cyclooxygenase inhibitor or prodrug thereof, and a pharmaceutically acceptable carrier, wherein the amount of the anti-muscarinic agent and the amount of the cyclooxygenase inhibitor together constitute a urinary incontinence condition effective amount of the anti-muscarinic agent and the cyclooxygenase inhibitor. For example, one of the many embodiments of the present invention is a combination comprising therapeutic dosages of an anti-muscarinic agent selected from Table 2 and a cyclooxygenase-2 selective inhibitor selected from Tables 3 and 5. A preferred embodiment of the present invention is a combination comprising therapeutic dosages of tolteridine and a tricyclic cyclooxygenase-2 selective inhibitor.

In yet another embodiment, the present invention comprises a therapeutic kit comprised of an amount of an anti-muscarinic agent in a dosage formulation and an amount of a cyclooxygenase inhibitor or prodrug in a separate dosage formulation wherein the amount of the anti-muscarinic agent and the amount of the cyclooxygenase inhibitor together constitute a urinary incontinence condition effective amount of the anti-muscarinic agent and the cyclooxygenase inhibitor.

Further scope of the applicability of the present invention will become apparent from the detailed description provided below. However, it should be understood that the following detailed description and examples, while indicating preferred embodiments of the invention, are given by way of illustration only, since various changes and modifications within the spirit and scope of the invention will become apparent from this detailed description to those skilled in the art.

### DETAILED DESCRIPTION OF THE PREFERRED EMBODIMENTS

The following detailed description is provided to aid those skilled in the art in practicing the present invention. Even so, this detailed description should not be construed to unduly limit the present invention, inasmuch as modifications and variations in the embodiments discussed herein can be made by those of ordinary skill in the art without departing from the spirit or scope of the present inventive discovery.

The contents of each of the references cited herein, including the contents of the references cited within these primary references, are herein incorporated by reference in their entirety.

### a. Definitions

The following definitions are provided in order to aid the reader in understanding the detailed description of the present invention:

The term "subject" as used herein refers to an animal, preferably a mammal, and particularly a human being, who has been the object of treatment, observation or experiment.

The terms "dosing" and "treatment" refer to any process, action, application, therapy, or the like, wherein a subject, and particularly a human being, is rendered medical aid with the object of improving the subject's condition, either directly or indirectly.

"Therapeutic compound" means a compound useful in the treatment of urinary incontinence conditions, including urge incontinence, stress incontinence, mixed incontinence, overactive bladder, neurogenic incontinence, detrusor hyperreflexia, suburethral diverticulitis, and urinary tract infection.

"Combination therapy" means the administration of two or more therapeutic compounds to treat a urinary incontinence condition, for example overactive bladder.

Such administration encompasses co-administration of these therapeutic compounds in a substantially simultaneous manner, such as in a single capsule having a fixed ratio of active ingredients or in multiple, separate capsules for each compound. In addition, such administration also encompasses use of each type of therapeutic compound in a sequential manner. In either case, the treatment regimen will provide beneficial effects of the drug combination in treating the incontinence condition.

The term "therapeutic combination" refers to the administered therapeutic compounds themselves and to any pharmaceutically acceptable carriers used to provide dosage forms such that the beneficial effect of each therapeutic compound is realized by the subject at the desired time, whether the compounds are administered substantially simultaneously or sequentially.

The phrase "therapeutically effective" is intended to qualify the combined amount of therapeutic compounds in the combination therapy. This combined amount will achieve the goal of avoiding or reducing or eliminating the urinary incontinence condition and/or interstitial cystitis condition.

The terms "cyclooxygenase-2 selective inhibitor" and "COX-2 selective inhibitor" interchangeably refer to a therapeutic compound which selectively inhibits the COX-2 isoform of the enzyme cyclooxygenase. COX-2 selectivity can be measured as a ratio of the in vitro or in vivo IC₅₀ value for inhibition of COX-1, divided by the IC₅₀ value for inhibition of COX-2. A COX-2 selective inhibitor is any inhibitor for which the ratio of COX-1 IC₅₀ to COX-2 IC₅₀ is greater than 1.

The term "prodrug" refers to a chemical compound that can be converted into a therapeutic compound by metabolic or simple chemical processes within the body of the subject. For example, a class of prodrugs of COX-2 inhibitors is described in US Patent No. 5,932,598, herein incorporated by reference.

### b. Combinations

The combinations of the present invention will have a number of uses. For example, through dosage adjustment and medical monitoring, the individual dosages of the therapeutic compounds used in the combinations of the present invention will be lower than are typical for dosages of the therapeutic compounds when used in monotherapy. The dosage lowering will provide advantages including reduction of side effects of the individual therapeutic compounds when compared to monotherapy. In addition, fewer side effects of the combination therapy compared with monotherapies will lead to greater patient compliance with therapy regimens.

### c. Anti-Muscarinic Agents

A large number and variety of anti-muscarinic agents are useful in the combinations and methods of the present invention. Some preferred anti-muscarinic agents are shown in Table 2.

**Table 2. Examples of Anti-Muscarinic Agents**

| **Compounds and Compound Classes** | **CAS Numbers for Specific and Representative Compounds** |
|---|---|
| Alvameline chloride | 23602-78-0 |
| Bethanechol chloride | 93957-54-1 |
| Darifenacin chloride | 75330-75-5 |
| Dicyclomine hydrochloride | 81093-37-0 |
| Emepronium chloride | 81093-37-0 |
| Hyoscyamine sulfate | 79902-63-9 |
| Imipramine hydrochloride | 134523-00-5 |
| Oxybutynin chloride | 145599-86-6 |
| S-Oxybutynin chloride | 132017-01-7 |
| Propantheline bromide | 147098-20-2 |
| Propiverine chloride | 141750-63-2 |
| Revatropate chloride | 132100-55-1 |
| Temiverine chloride | 73573-88-3 |
| Terodiline chloride | 147098-18-8 |
| Tolteridine tartrate | 147098-20-2 |
| Trospium chloride | 129829-03-4 |
| Vamicamide Chloride | 141750-63-2 |
| AH-9700 | 148966-78-3 |
| FK-584 | 125894-01-1 |
| J-104135 | 157058-13-4 |
| KRP-197 | 157555-28-7 |
| YM-905 | 64405-40-9 |
| YM-46303 | 129829-03-4 |

### d. Cyclooxygenase Inhibitors

The present invention provides that treatment of a subject with one or more cyclooxygenase inhibitors, alone or in combination with an anti-muscarinic agent, results in the effective treatment or prophylaxis of urinary incontinence conditions or interstitial cystitis. In one embodiment, the method comprises treating the subject with a urinary incontinence condition-effective amount of a cyclooxygenase inhibitor or a prodrug thereof. In another embodiment the method comprises treating a subject with an amount of an anti-muscarinic agent and an amount of a cyclooxygenase inhibitor or prodrug thereof, wherein the amount of the anti-muscarinic agent and the amount of the cyclooxygenase inhibitor together comprise a urinary incontinence condition treating or prophylactic-effective amount of the anti-muscarinic agent and the cyclooxygenase inhibitor.

For example, one of the many embodiments of the present invention is a combination therapy comprising a therapeutic amount of an anti-muscarinic agent and a therapeutic amount of a cyclooxygenase-inhibiting non-steroidal anti-inflammatory drug (NSAID). Examples of cyclooxygenase-inhibiting NSAIDs include the well-known compounds aspirin, indomethacin, sulindac, etodolac, mefenamic acid, tolmetin, ketorolac, diclofenac, ibuprofen, naproxen, fenoprofen, ketoprofen, oxaprozin, flurbiprofen, nitroflurbiprofen, piroxicam, tenoxicam, phenylbutazone, apazone, or nimesulide or a pharmaceutically acceptable salt or derivative or prodrug thereof. In a preferred embodiment of the invention the NSAID is selected from the group comprising indomethacin, ibuprofen, naproxen, flurbiprofen or nitroflurbiprofen. In another preferred embodiment of the invention the NSAID is selected from the group comprising indomethacin, naproxen, flurbiprofen or nitroflurbiprofen. In a still more preferred embodiment of the invention the NSAID is nitroflurbiprofen. Some of the NSAIDs listed above may inhibit cyclooxygenase-2 to a different extent in vivo or in vitro than they inhibit cyclooxygenase-1.

In another embodiment of the invention the cyclooxygenase inhibitor can be a cyclooxygenase-2 selective inhibitor. The terms "cyclooxygenase-2 selective inhibitor" and "COX-2 selective inhibitor" interchangeably refer to a therapeutic compound which selectively inhibits the COX-2 isoform of the enzyme cyclooxygenase. In practice, COX-2 selectivity varies depending on the conditions under which the test is performed and on the inhibitors being tested. However, for the purposes of this patent, COX-2 selectivity can be measured as a ratio of the *in vitro* or *in vivo* IC₅₀ value for inhibition of COX-1, divided by the IC₅₀ value for inhibition of COX-2. A COX-2 selective inhibitor is any inhibitor for which the ratio of COX-1 IC₅₀ to COX-2 IC₅₀ is greater than 1, preferably greater than 5, more preferably greater than 10, still more preferably greater than 50, and more preferably still greater than 100.

The term "prodrug" refers to a chemical compound that can be converted into a therapeutic compound by metabolic or simple chemical processes within the body of the subject. For example, a class of prodrugs of COX-2 inhibitors is described in US Patent No. 5,932,598, herein incorporated by reference.

In one embodiment of the invention the COX-2 selective inhibitor is meloxicam, Formula A-1 (CAS registry number 71125-38-7) or a pharmaceutically acceptable salt or derivative or prodrug thereof.

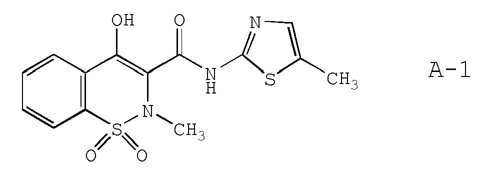

In another embodiment of the invention the cyclooxygenase-2 selective inhibitor is the COX-2 selective inhibitor RS-57067, 6-[[5-(4-chlorobenzoyl)-1,4-dimethyl-1H-pyrrol-2-yl]methyl]-3(2H)-pyridazinone, Formula A-2 (CAS registry number 179382-91-3) or a pharmaceutically acceptable salt or derivative or prodrug thereof.

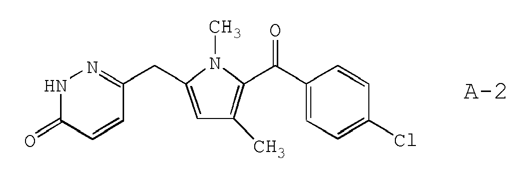

In another embodiment of the invention the cyclooxygenase-2 selective inhibitor is the COX-2 selective inhibitor ABT-963, 2-(3,4-difluorophenyl)-4-(3-hydroxy-3-methylbutoxy)-5-[4-(methylsulfonyl)phenyl]-(9Cl)-3(2H)-pyridazinone, Formula A-3 (CAS registry number 266320-83-6 or a pharmaceutically acceptable salt or derivative or prodrug thereof.

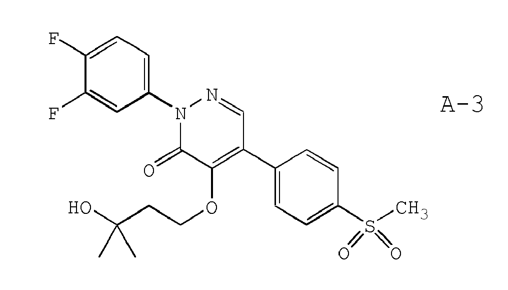

In another embodiment of the invention the cyclooxygenase-2 selective inhibitor is the COX-2 selective inhibitor COX-189, Formula A-4 (CAS registry number 346670-74-4) or a pharmaceutically acceptable salt or derivative or prodrug thereof.

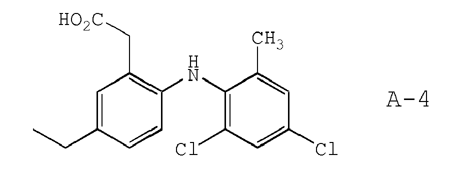

In another embodiment of the invention the cyclooxygenase-2 selective inhibitor is the COX-2 selective inhibitor NS-398, N-(2-cyclohexyl-4-nitrophenyl)methanesulfonamide, Formula A-5 (CAS registry number 123653-11-2) or a pharmaceutically acceptable salt or derivative or prodrug thereof.

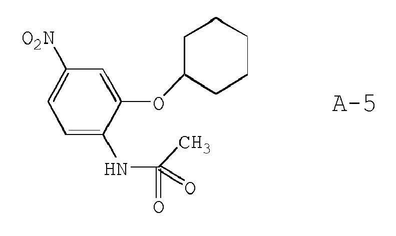

In a preferred embodiment of the invention the cyclooxygenase-2 selective inhibitor is a COX-2 selective inhibitor of the chromene structural class that is a substituted benzopyran or a substituted benzopyran analog selected from the group consisting of substituted benzothiopyrans, dihydroquinolines, or dihydronaphthalenes having the general Formula II shown below and possessing, by way of example and not limitation, the structures disclosed in Table 3, including the diastereomers, enantiomers, racemates, tautomers, salts, esters, amides and prodrugs thereof.

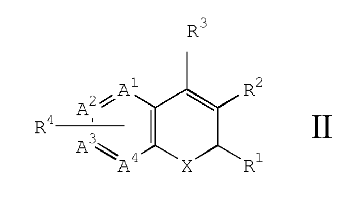

**Table 3. Examples of Chromene COX-2 Selective Inhibitors as Embodiments**

| **Compound Number** | **Structural Formula** |
|---|---|
| A-6 | 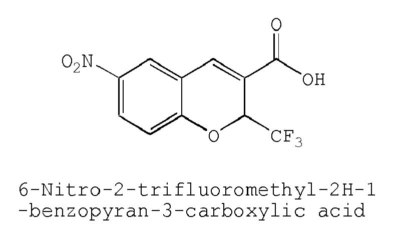 |
| A-7 | 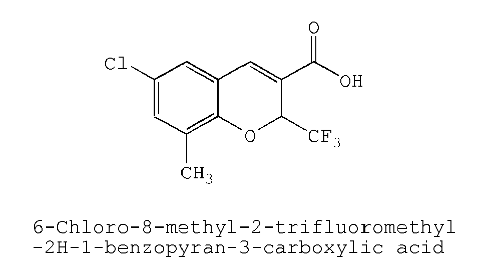 |
| A-8 | 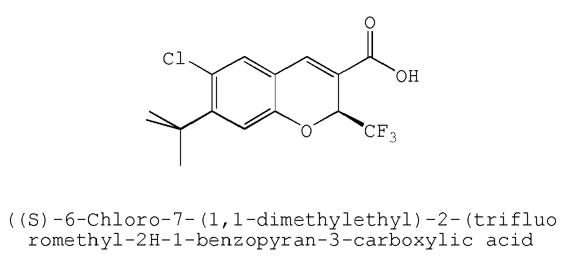 |
| A-9 | 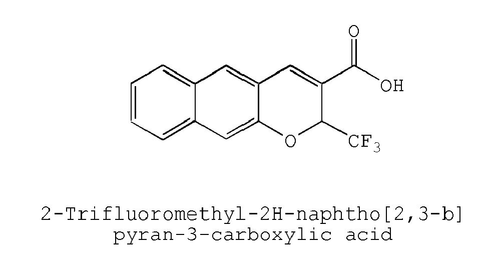 |
| A-10 | 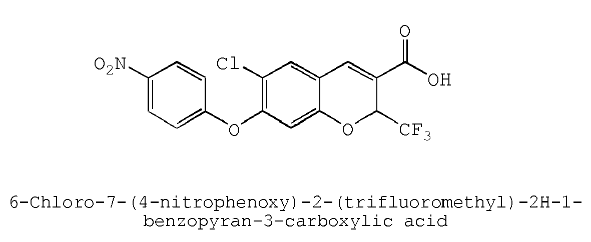 |
| A-11 | 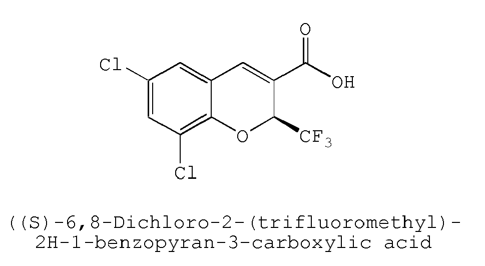 |
| A-12 | 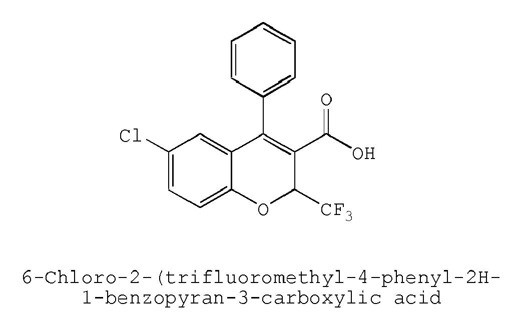 |
| A-13 | 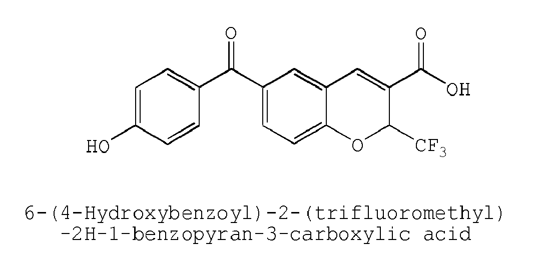 |
| A-14 | 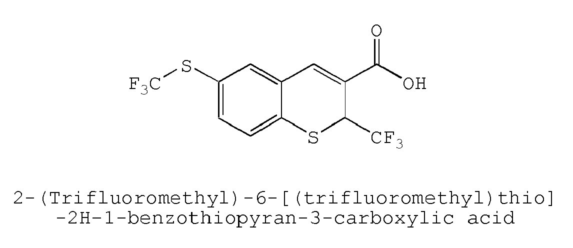 |
| A-15 | 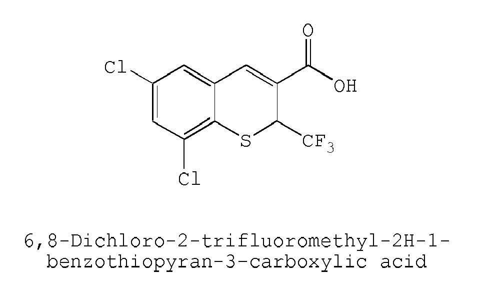 |
| A-16 | 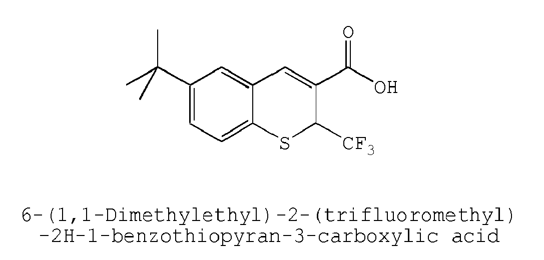 |
| A-17 | 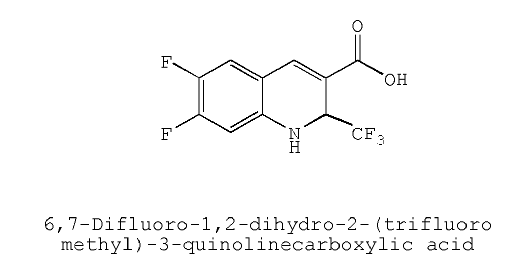 |
| A-18 | 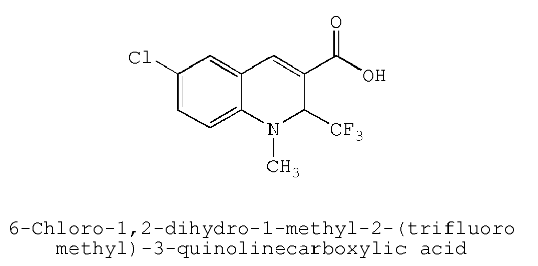 |
| A-19 | 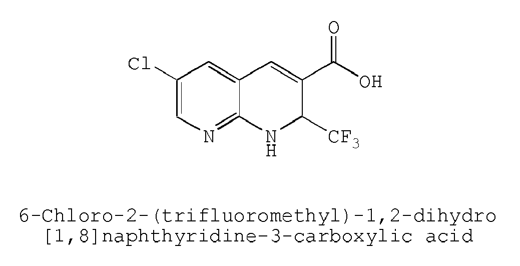 |
| A-20 | 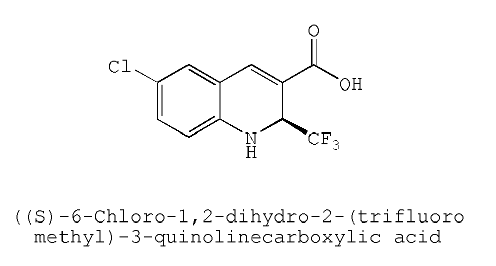 |

The individual patent documents referenced in Table 4 below describe the preparation of the aforementioned COX-2 inhibitors of Table 3 and each patent document is herein individually incorporated by reference.

**Table 4. References for Preparation of Chromene COX-2 Inhibitors**

| **Compound Number** | **Patent Reference** |
|---|---|
| A-6 | US 6,077,850; example 37 |
| A-7 | US 6,077,850; example 38 |
| A-8 | US 6,077,850; example 68 |
| A-9 | US 6,034,256; example 64 |
| A-10 | US 6,077,850; example 203 |
| A-11 | US 6,034,256; example 175 |
| A-12 | US 6,077,850; example 143 |
| A-13 | US 6,077,850; example 98 |
| A-14 | US 6,077,850; example 155 |
| A-15 | US 6,077,850; example 156 |
| A-16 | US 6,077,850; example 147 |
| A-17 | US 6,077,850; example 159 |
| A-18 | US 6,034,256; example 165 |
| A-19 | US 6,077,850; example 174 |
| A-20 | US 6,034,256; example 172 |

In a more preferred embodiment of the invention the cycloxygenase-2 selective inhibitor is the substituted benzopyran (S)-6,8-dichloro-2-(trifluoromethyl)-2H-1-benzopyran-3-carboxylic acid (SD-8381), Formula A-11, or a pharmaceutically acceptable salt or derivative or prodrug thereof.

In a further preferred embodiment of the invention the cyclooxygenase inhibitor is selected from the class of tricyclic cyclooxygenase-2 selective inhibitors represented by the general structure of Formula III 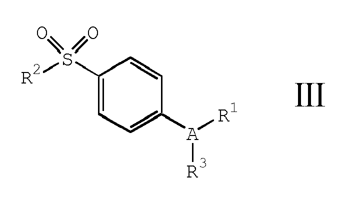 wherein A is a substituent selected from partially unsaturated or unsaturated heterocyclyl and partially unsaturated or unsaturated carbocyclic rings;
wherein R¹ is at least one substituent selected from heterocyclyl, cycloalkyl, cycloalkenyl and aryl, wherein R¹ is optionally substituted at a substitutable position with one or more radicals selected from alkyl, haloalkyl, cyano, carboxyl, alkoxycarbonyl, hydroxyl, hydroxyalkyl, haloalkoxy, amino, alkylamino, arylamino, nitro, alkoxyalkyl, alkylsulfinyl, halo, alkoxy and alkylthio;
wherein R² is methyl or amino; and
wherein R³ is a radical selected from hydrido, halo, alkyl, alkenyl, alkynyl, oxo, cyano, carboxyl, cyanoalkyl, heterocyclyloxy, alkyloxy, alkylthio, alkylcarbonyl, cycloalkyl, aryl, haloalkyl, heterocyclyl, cycloalkenyl, aralkyl, heterocyclylalkyl, acyl, alkylthioalkyl, hydroxyalkyl, alkoxycarbonyl, arylcarbonyl, aralkylcarbonyl, aralkenyl, alkoxyalkyl, arylthioalkyl, aryloxyalkyl, aralkylthioalkyl, aralkoxyalkyl, alkoxyaralkoxyalkyl, alkoxycarbonylalkyl, aminocarbonyl, aminocarbonylalkyl, alkylaminocarbonyl, N-arylaminocarbonyl, N-alkyl-N-arylaminocarbonyl, alkylaminocarbonylalkyl, carboxyalkyl, alkylamino, N-arylamino, N-aralkylamino, N-alkyl-N-aralkylamino, N-alkyl-N-arylamino, aminoalkyl, alkylaminoalkyl, N-arylaminoalkyl, N-aralkylaminoalkyl, N-alkyl-N-aralkylaminoalkyl, N-alkyl-N-arylaminoalkyl, aryloxy, aralkoxy, arylthio, aralkylthio, alkylsulfinyl, alkylsulfonyl, aminosulfonyl, alkylaminosulfonyl, N-arylaminosulfonyl, arylsulfonyl, N-alkyl-N-arylaminosulfonyl; or a pharmaceutically acceptable salt or derivative or prodrug thereof.

In a still more preferred embodiment of the invention the cyclooxygenase-2 selective inhibitor represented by the above Formula III is selected from the group of compounds, illustrated in Table 5, consisting of celecoxib (A-21), valdecoxib (A-22), deracoxib (A-23), rofecoxib (A-24), etoricoxib (MK-663; A-25), JTE-522 (A-26), or a pharmaceutically acceptable salt or derivative or prodrug thereof.

In an even more preferred embodiment of the invention the COX-2 selective inhibitor is selected from the group consisting of celecoxib, rofecoxib and etoricoxib.

**Table 5. Examples of Tricyclic COX-2 Selective Inhibitors as Embodiments**

| **Compound Number** | **Structural Formula** |
|---|---|
| A-21 | 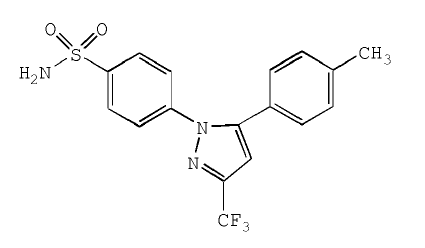 |
| A-22 | 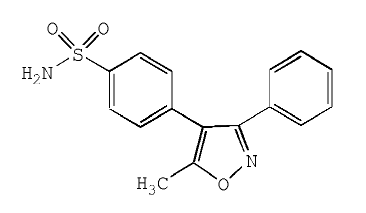 |
| A-23 | 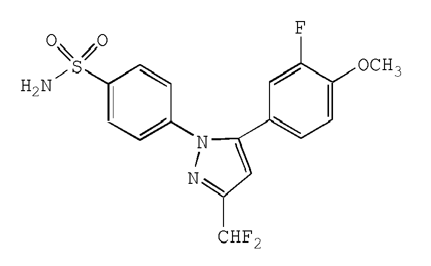 |
| A-24 | 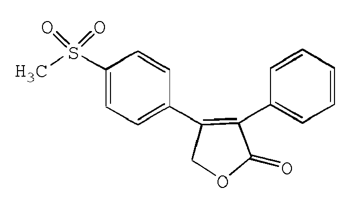 |
| A-25 | 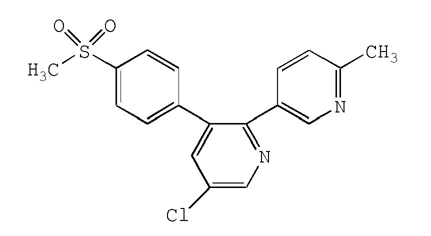 |
| A-26 | 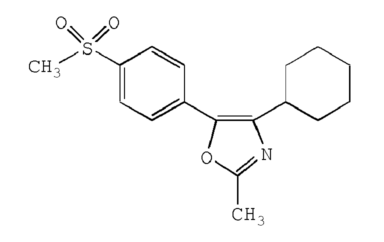 |

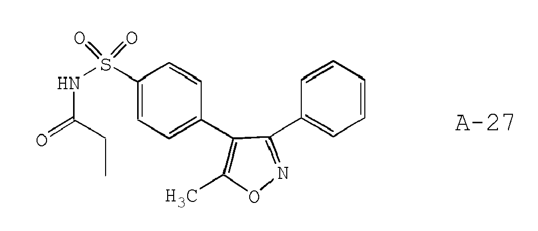

The individual patent documents referenced in Table 6 below describe the preparation of the aforementioned cyclooxygenase-2 selective inhibitors A-21 through A-27 and each patent document is herein incorporated by reference.

**Table 6. References for Preparation of Tricyclic COX-2 Inhibitors and Prodrugs**

| **Compound Number** | **Patent Reference** |
|---|---|
| A-21 | US 5,466,823 |
| A-22 | US 5,633,272 |
| A-23 | US 5,521,207 |
| A-24 | US 5,840,924 |
| A-25 | WO 98/03484 |
| A-26 | WO 00/25779 |
| A-27 | US 5,932,598 |

### e. Dosages, Formulations, and Routes of Administration

Many of the compounds useful in the present invention can have at least two asymmetric carbon atoms, and therefore include racemates and stereoisomers, such as diastereomers and enantiomers, in both pure form and in admixture. Such stereoisomers can be prepared using conventional techniques, either by reacting enantiomeric starting materials, or by separating isomers of compounds of the present invention. Isomers may include geometric isomers, for example *cis*-isomers or *trans-*isomers across a double bond. All such isomers are contemplated among the compounds useful in the present invention. The compounds useful in the present invention also include tautomers. The compounds useful in the present invention as discussed below include their salts, solvates and prodrugs.

The combinations of the present invention can be administered for the treatment of urinary incontinence conditions by any means, preferably oral, that produce contact of these compounds with their site of action in the body, for example in the bladder of a mammal, e.g., a human. For the treatment of the conditions referred to above, the compounds useful in the combinations and methods of the present invention can be used as the compound *per se.* Pharmaceutically acceptable salts are particularly suitable for medical applications because of their greater aqueous solubility relative to the parent compound. Such salts must clearly have a pharmaceutically acceptable anion or cation. Suitable pharmaceutically acceptable acid addition salts of the compounds of the present invention when possible include those derived from inorganic acids, such as hydrochloric, hydrobromic, phosphoric, metaphosphoric, nitric, sulfonic, and sulfuric acids, and organic acids such as acetic, benzenesulfonic, benzoic, citric, ethanesulfonic, fumaric, gluconic, glycolic, isothionic, lactic, lactobionic, maleic, malic, methanesulfonic, succinic, toluenesulfonic, tartaric, and trifluoroacetic acids. The chloride salt is particularly preferred for medical purposes. Suitable pharmaceutically acceptable base salts include ammonium salts, alkali metal salts such as sodium and potassium salts, and alkaline earth salts such as magnesium and calcium salts.

The anions useful in the present invention are, of course, also required to be pharmaceutically acceptable and are also selected from the above list.

The compounds useful in the present invention can be presented with an acceptable carrier in the form of a pharmaceutical composition. The carrier must, of course, be acceptable in the sense of being compatible with the other ingredients of the composition and must not be deleterious to the recipient. The carrier can be a solid or a liquid, or both, and is preferably formulated with the compound as a unit-dose composition, for example, a tablet, which can contain from 0.05% to 95% by weight of the active compound. Other pharmacologically active substances can also be present, including other compounds of the present invention. The pharmaceutical compositions of the invention can be prepared by any of the well-known techniques of pharmacy, consisting essentially of admixing the components.

These compounds can be administered by any conventional means available for use in conjunction with pharmaceuticals, either as individual therapeutic compounds or as a combination of therapeutic compounds.

The amount of compound which is required to achieve the desired biological effect will, of course, depend on a number of factors such as the specific compound chosen, the use for which it is intended, the mode of administration, and the clinical condition of the recipient.

In general, a total daily dose of a anti-muscarinic agent can be in the range of from about 0.01 to about 20 mg/day, preferably from about 0.1 to about 10 mg/day, more preferably from about 0.5 to about 5.0 mg/day.

A total daily dose of a cyclooxygenase-2 selective inhibitor can be in the range of from about 0.3 to about 100 mg/kg body weight/day, preferably from about 1 to about 50 mg/kg body weight/day, more preferably from about 3 to about 10 mg/kg body weight/day.

The daily doses described in the preceding paragraphs for the various therapeutic compounds can be administered to the patient in a single dose, or in proportionate multiple subdoses. Subdoses can be administered 2 to 6 times per day. Doses can be in sustained release form effective to obtain desired results.

In the case of pharmaceutically acceptable salts, the weights indicated above refer to the weight of the acid equivalent or the base equivalent of the therapeutic compound derived from the salt.

Oral delivery of the individual therapeutic compounds and combinations of the present invention can include formulations, as are well known in the art, to provide prolonged or sustained delivery of the drug to the gastrointestinal tract by any number of mechanisms. These include, but are not limited to, osmotic tablets, gel matrix tablets, coated beads, and the like. Other mechanisms include pH sensitive release from the dosage form based on the changing pH of the small intestine, slow erosion of a tablet or capsule, retention in the stomach based on the physical properties of the formulation, bioadhesion of the dosage form to the mucosal lining of the intestinal tract, or enzymatic release of the active drug from the dosage form. For some of the therapeutic compounds useful in the present invention (e.g., anti-muscarinic agents), the intended effect is to extend the time period over which the active drug molecule is delivered to the desired site of action (e.g., the bladder) by manipulation of the dosage form, while at the same time minimizing delivery to undesired sites of action (e.g., the oral cavity).
Thus, enteric-coated and enteric-coated controlled release formulations are within the scope of the present invention. Suitable enteric coatings include, but are not limited to, cellulose acetate phthalate, polyvinylacetate phthalate, hydroxypropylmethylcellulose phthalate and anionic polymers of methacrylic acid and methacrylic acid methyl ester.

The individual therapeutic compounds and combinations of the present invention can be delivered orally either in a solid, in a semi-solid, or in a liquid form. When in a liquid or in a semi-solid form, the compounds and combinations of the present invention can, for example, be in the form of a liquid, syrup, or contained in a gel capsule (e.g., a gel cap).

The compounds and combinations of the present invention can also be administered by means of a transdermal patch using conventional technology in order to reduce side effects and obtain improved subject compliance. The compounds and combinations of the present invention can also be delivered to the bladder intravesically in the form of instilled solutions.

When administered intravenously, the dose for an anti-muscarinic agent can, for example, be in the range of from about 0.01 mg to about 20 mg/day, preferably from about 0.1 to about 10 mg/day, more preferably from about 0.5 to about 5.0 mg/day.

For a cyclooxygenase-2 selective inhibitor the intravenously administered dose can, for example, be in the range of from about 0.003 to about 1.0 mg/kg body weight/day, preferably from about 0.01 to about 0.75 mg/kg body weight/day, more preferably from about 0.1 to about 0.6 mg/kg body weight/day.

The dose of any of these therapeutic compounds can be conveniently administered as an infusion of from about 10 ng/kg body weight to about 100 ng/kg body weight per minute. Infusion fluids suitable for this purpose can contain, for example, from about 0.1 ng to about 10 mg, preferably from about 1 ng to about 10 mg per milliliter. Unit doses can contain, for example, from about 1 mg to about 10 g of the compound of the present invention. Thus, ampoules for injection can contain, for example, from about 1 mg to about 100 mg.

Pharmaceutical compositions according to the present invention include those suitable for oral, transdermal, intravesical, rectal, topical, buccal (e.g., sublingual), and parenteral (e.g., subcutaneous, intramuscular, intradermal, or intravenous) administration, although the most suitable route in any given case will depend on the nature and severity of the condition being treated and on the nature of the particular compound which is being used. In most cases, the preferred route of administration is oral.

Pharmaceutical compositions suitable for oral administration can be presented in discrete units, such as capsules, cachets, lozenges, or tablets, each containing a predetermined amount of at least one therapeutic compound useful in the present invention; as a powder or granules; as a solution or a suspension in an aqueous or non-aqueous liquid; or as an oil-in-water or water-in-oil emulsion. As indicated, such conmpositions can be prepared by any suitable method of pharmacy which includes the step of bringing into association the active compound(s) and the carrier (which can constitute one or more accessory ingredients). In general, the compositions are prepared by uniformly and intimately admixing the active compound with a liquid or finely divided solid carrier, or both, and then, if necessary, shaping the product. For example, a tablet can be prepared by compressing or molding a powder or granules of the compound, optionally with one or more accessory ingredients. Compressed tablets can be prepared by compressing, in a suitable machine, the compound in a free-flowing form, such as a powder or granules optionally mixed with a binder, lubricant, inert diluent and/or surface active/dispersing agent(s). Molded tablets can be made by molding, in a suitable machine, the powdered compound moistened with an inert liquid diluent.

Pharmaceutical compositions suitable for buccal (sub-lingual) administration include lozenges comprising a compound of the present invention in a flavored base, usually sucrose, and acacia or tragacanth, and pastilles comprising the compound in an inert base such as gelatin and glycerin or sucrose and acacia.

Pharmaceutical compositions suitable for parenteral administration conveniently comprise sterile aqueous preparations of a compound of the present invention. These preparations are preferably administered intravenously, although administration can also be effected by means of subcutaneous, intramuscular, or intradermal injection. Such preparations can conveniently be prepared by admixing the compound with water and rendering the resulting solution sterile and isotonic with the blood. Injectable compositions according to the invention will generally contain from 0.1 to 5% w/w of a compound disclosed herein.

Pharmaceutical compositions suitable for rectal administration are preferably presented as unit-dose suppositories. These can be prepared by admixing a compound of the present invention with one or more conventional solid carriers, for example, cocoa butter, and then shaping the resulting mixture.

Pharmaceutical compositions suitable for topical application to the skin preferably take the form of an ointment, cream, lotion, paste, gel, spray, aerosol, or oil. Carriers which can be used include petroleum jelly (e.g., Vaseline), lanolin, polyethylene glycols, alcohols, and combinations of two or more thereof. The active compound is generally present at a concentration of from 0.1 to 50% w/w of the composition, for example, from 0.5 to 2%.

Transdermal administration is also possible.

Pharmaceutical compositions suitable for transdermal administration can be presented as discrete patches adapted to remain in intimate contact with the epidermis of the recipient for a prolonged period of time. Such patches suitably contain a compound of the present invention in an optionally buffered, aqueous solution, dissolved and/or dispersed in an adhesive, or dispersed in a polymer. A suitable concentration of the active compound is about 1% to 35%, preferably about 3% to 15%. As one particular possibility, the compound can be delivered from the patch by electrotransport or iontophoresis, for example, as described in Pharmaceutical Research, 3, 318 (1986), herein incorporated by reference.

In any case, the amount of active ingredient that can be combined with carrier materials to produce a single dosage form to be administered will vary depending upon the host treated and the particular mode of administration.

The solid dosage forms for oral administration including capsules, tablets, pills, powders, gel caps, and granules noted above comprise one or more compounds useful in the present invention admixed with at least one inert diluent such as sucrose, lactose, or starch. Such dosage forms may also comprise, as in normal practice, additional substances other than inert diluents, e.g., lubricating agents such as magnesium stearate or solubilizing agents such as cyclodextrins. In the case of capsules, tablets, powders, granules, gel caps, and pills, the dosage forms may also comprise buffering agents. Tablets and pills can additionally be prepared with enteric coatings.

Liquid dosage forms for oral administration can include pharmaceutically acceptable emulsions, solutions, suspensions, syrups, and elixirs containing inert diluents commonly used in the art, such as water.

Such compositions may also comprise adjuvants, such as wetting agents, emulsifying and suspending agents, and sweetening, flavoring, and perfuming agents.

Injectable preparations, for example, sterile injectable aqueous or oleaginous suspensions may be formulated according to the known art using suitable dispersing or setting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Pharmaceutically acceptable carriers encompass all the foregoing and the like.

In combination therapy, administration of two or more of the therapeutic agents useful in the present invention may take place sequentially in separate formulations, or may be accomplished by simultaneous administration in a single formulation or separate formulations. Administration may be accomplished by oral route, or by intravenous, intramuscular, or subcutaneous injections. The formulation may be in the form of a bolus, or in the form of aqueous or non-aqueous isotonic sterile injection solutions or suspensions. These solutions and suspensions may be prepared from sterile powders or granules having one or more pharmaceutically-acceptable carriers or diluents, or a binder such as gelatin or hydroxypropylmethyl cellulose, together with one or more of a lubricant, preservative, surface active or dispersing agent.

For oral administration, the pharmaceutical composition may be in the form of, for example, a tablet, capsule, suspension, or liquid. Capsules, tablets, etc., can be prepared by conventional methods well known in the art. The pharmaceutical composition is preferably made in the form of a dosage unit containing a particular amount of the active ingredient or ingredients. Examples of dosage units are tablets or capsules. These may with advantage contain one or more therapeutic compound in an amount described above.

The active ingredients may also be administered by injection as a composition wherein, for example, saline, dextrose, or water may be used as a suitable carrier. A suitable daily dose of each active therapeutic compound is one that achieves the same blood serum level as produced by oral administration as described above.

The therapeutic compounds may further be administered by any combination of oral/oral, oral/parenteral, or parenteral/parenteral route.

Pharmaceutical compositions for use in the treatment methods of the present invention may be administered in oral form or by intravenous administration. Oral administration of the combination therapy is preferred. Dosing for oral administration may be with a regimen calling for single daily dose, or for a single dose every other day, or for multiple, spaced doses throughout the day. The therapeutic compounds which make up the combination therapy may be administered simultaneously, either in a combined dosage form or in separate dosage forms intended for substantially simultaneous oral administration. The therapeutic compounds which make up the combination therapy may also be administered sequentially, with either therapeutic compound being administered by a regimen calling for two-step ingestion. Thus, a regimen may call for sequential administration of the therapeutic compounds with spaced-apart ingestion of the separate, active agents. The time period between the multiple ingestion steps may range from a few minutes to several hours, depending upon the properties of each therapeutic compound such as potency, solubility, bioavailability, plasma half-life and kinetic profile of the therapeutic compound, as well as depending upon the effect of food ingestion and the age and condition of the patient. Circadian variation of the target molecule concentration may also determine the optimal dose interval. The therapeutic compounds of the combined therapy whether administered simultaneously, substantially simultaneously, or sequentially, may involve a regimen calling for administration of one therapeutic compound by oral route and another therapeutic compound by intravenous route. Whether the therapeutic compounds of the combined therapy are administered by oral or intravenous route, separately or together, each such therapeutic compound will be contained in a suitable pharmaceutical formulation of pharmaceutically-acceptable excipients, diluents or other formulations components. Examples of suitable pharmaceutically-acceptable formulations containing the therapeutic compounds for oral administration are given above.

### g. Treatment Regimen

The dosage regimen to prevent, give relief from, or ameliorate urinary incontinence is selected in accordance with a variety of factors. These include the type, age, weight, sex, diet, and medical condition of the patient, the severity of the disease, the route of administration, pharmacological considerations such as the activity, efficacy, pharmacokinetics and toxicology profiles of the particular compound employed, whether a drug delivery system is utilized, and whether the compound is administered as part of a drug combination.

Thus, the dosage regimen actually employed may vary widely and therefore deviate from the preferred dosage regimen set forth above.

Initial treatment of a patient suffering from overactive bladder can begin with the dosages indicated above. Treatment should generally be continued as necessary over a period of several weeks to several months or years or until urinary incontinence has been controlled or eliminated. Patients undergoing treatment with the compounds or combinations disclosed herein can be routinely monitored by observing micturition patterns to determine the effectiveness of the combination therapy. In this way, the treatment regimen/dosing schedule can be rationally modified over the course of therapy so that the lowest amount of the therapeutic compounds which together exhibit satisfactory effectiveness is administered, and so that administration is continued only so long as is necessary to successfully treat the urinary incontinence condition. A potential advantage of the combination therapy disclosed herein may be reduction of the amount of any individual therapeutic compound, or all therapeutic compounds, effective in treating overactive bladder.

The embodiments of the present invention can comprise a combination therapy using two or more of the therapeutic compounds described or incorporated herein.

The combination therapy can comprise two or more therapeutic compounds having a similar effect from different classes of chemistry, e.g., benzopyran cyclooxygenase-2 selective inhibitors can be therapeutically combined with tricyclic cyclooxygenase-2 selective inhibitors. Therapeutic combinations can also comprise more than two therapeutic compounds. Alternatively, two or more compounds from the same therapeutic class of chemistry can comprise the therapy, e.g. a combination therapy comprising two or more anti-muscarinic agents or two or more tricyclic cyclooxygenase-2 selective inhibitors.

### h. Kits

The present invention further comprises kits that are suitable for use in performing the methods of treatment and/or prophylaxis described above. In one embodiment, the kit contains a first dosage form comprising one or more anti-muscarinic agents identified in Table 2 and a second dosage form comprising a cyclooxygenase-inhibiting non-steroidal anti-inflammatory drug (NSAID) in quantities sufficient to carry out the methods of the present invention. In a more preferred embodiment the kit contains a first dosage form comprising one or more of the anti-muscarinic agents identified in Table 2 and a second dosage form comprising a COX-2 selective inhibitor in quantities sufficient to carry out the methods of the present invention. In a still more preferred embodiment the kit contains a first dosage form inhibitors identified in Table 2 and a second dosage form comprising a COX-2 selective chromene inhibitor identified in Table 3. In an even more highly preferred embodiment the kit contains a first dosage form comprising one or more of the anti-muscarinic agents identified in Table 2 and a second dosage form comprising a COX-2 selective tricyclic inhibitor identified in Table 5. In a particularly preferred embodiment the kit contains a first dosage form comprising the anti-muscarinic agents tolteridine tartrate and a second dosage form comprising either celecoxib (A-21) or rofecoxib (A-24).

### i. Biological Assays of Utility

The utility of the combinations of the present invention can be shown by the following assays. Assays are performed *in vitro* and in animal models using procedures well recognized to show the utility of the present invention.

### In Vitro Assay of Compounds That Inhibit Recombinant COX-1 and/or COX-2 Activity

### a. Preparation of Recombinant COX Baculoviruses

Recombinant COX-1 and COX-2 are prepared as described by Gierse et al. (J. Biochem., 305, 479-484 (1995), herein incorporated by reference). A 2.0 kb fragment containing the coding region of either human or murine COX-1 or human or murine COX-2 is cloned into a BamH1 site of the baculovirus transfer vector pVL1393 (Invitrogen) to generate the baculovirus transfer vectors for COX-1 and COX-2 in a manner similar to the method of D.R. O'Reilly et al. (Baculovirus Expression Vectors: A Laboratory Manual (1992), herein incorporated by reference). Recombinant baculoviruses are isolated by transfecting 4 pg of baculovirus transfer vector DNA into SF9 insect cells (2x10⁸) along with 200 ng of linearized baculovirus plasmid DNA by the calcium phosphate method (M.D. Summers and G.E Smith, A Manual of Methods for Baculovirus Vectors and Insect Cell Culture Procedures, Texas Agric. Exp. Station Bull. 1555 (1987)). Recombinant viruses are purified by three rounds of plaque purification, and high-titer (10⁷-10⁸ pfu/mL) stocks of virus were prepared. For large-scale production, SF9 insect cells are infected in 10-liter fermentors (0.5x10⁶/mL) with the recombinant baculovirus stock such that the multiplicity of the infection was 0.1. After 72 hours the cells are centrifuged, and the cell pellet homogenized in Tris/Sucrose (50 mM: 25%, pH 8.0) containing 1% 3-[(3)-cholamidopropyl)dimethylammonio]-1-propanesulfonate (CHAPS). The homogenate is centrifuged at 10,000 x G for 30 minutes, and the resulting supernatant is stored at -80° C before being assayed for COX activity.

### b. Assay for COX-1 and COX-2 Activity

COX activity is assayed as PGE₂ formed/jg protein/time using an ELISA to detect the prostaglandin released. CHAPS-solubilized insect cell wall membranes containing the appropriate COX enzyme are incubated in a potassium phosphate buffer (50 mM, pH 8.0) containing epinephrine, phenol, and heme with the addition of arachidonic acid (10 uM). Compounds are pre-incubated with the enzyme for 10-20 minutes prior to the addition of arachidonic acid. Any reaction between the arachidonic acid and the enzyme is stopped after 10 minutes at 37° C/room temperature by transferring 40 uL of reaction mix into 160 uL ELISA buffer and 25 uM indomethacin. The PGE₂ formed is measured by standard ELISA technology (Cayman Chemical) .

### c. Rapid assay for COX-1 and COX-2 Activity

COX activity is assayed as PGE₂ formed/ug protein/time using an ELISA to detect the prostaglandin released. CHAPS-solubilized insect cell wall membranes containing the appropriate COX enzyme are incubated in a potassium phosphate buffer (50 mM potassium phosphate, pH 7.5, 300uu epinephrine, 2 uM phenol, 1 uM heme) with the addition of 20 uL of 100 uM arachidonic acid (10 uM). Compounds are pre-incubated with the enzyme for 10 minutes at 37° C prior to the addition of arachidonic acid. Any reaction between the arachidonic acid and the enzyme is stopped after 2 minutes at 37° C/room temperature by transferring 40 uL of reaction mix into 160 uL ELISA buffer and 25 uM indomethacin. The PGE₂ formed is measured by standard ELISA technology (Cayman Chemical).

### In Vivo Assay of Action Against Acetylcholine-Induced Bladder Contraction

Male Sprague-Dawley rats weighing about 300 g are immobilized on their backs under intraperitoneal anesthesia with urethane and alpha-chloralose, and each animal's bladder is exposed by midline abdominal incision. A polyethylene tube filled with physiological saline is inserted into the top part of the bladder, and intracystic pressure is measured. A venous cannula for drug administration is inserted into the femoral vein, and a solution of 10 ug/kg of acetylcholine is administered at 10-minute intervals in order to induce bladder contraction.

The stomach is subjected to midline incision, and test compounds are intraduodenally dosed using an injection needle. The action of test compounds in inhibiting bladder contraction is observed for 120 minutes after dosing. Bladder contraction is measured as a difference in intracystic pressure before and after each administration of acetylcholine. Bladder contraction before dosing of a test compound or combination of compounds is designated as the pre-dosing value, and contraction after dosing is compared with the pre-dosing value, allowing a 50% inhibitory dose to be calculated for each test sample.

## Claims

1. A cyclooxygenase-2 selective inhibitor or pharmaceutically acceptable salt or prodrug thereof in combination with an anti-muscarinic agent or a pharmaceutically acceptable salt thereof for use in the treatment or prophylaxis of interstitial cystitis in a subject in need of such treatment or prophylaxis.

2. The use as claimed in claim 1, wherein the cyclooxygenase inhibitor is a non-steroidal anti-inflammatory drug.

3. The use as claimed in claim 1, wherein the cyclooxygenase-2 selective inhibitor is selected from the group consisting of meloxicam, RS-57067, ABT-963, COX-189, NS-398, celecoxib, valdecoxib, deracoxib, rofecoxib, etoricoxib (MK-663), and JTE-522, or a pharmaceutically acceptable salt or prodrug thereof.

4. The use as claimed in claim 3, wherein the cyclooxygenase-2 selective inhibitor is celecoxib.

5. The use as claimed in claim 3, wherein the cyclooxygenase-2 selective inhibitor is rofecoxib.

6. The use as claimed in claim 3, wherein parecoxib is employed as a prodrug and source of the cyclooxygenase-2 selective inhibitor valdecoxib.

7. The use as claimed in claim 1, wherein the cyclooxygenase-2 selective inhibitor is a substituted benzopyran or a pharmaceutically acceptable salt or prodrug thereof.

8. The use as claimed in claim 1, wherein the cyclooxygenase-2 selective inhibitor is a substituted benzopyran analog selected from the group consisting of substituted benzothiopyrans, dihydroquinolines, and dihydronaphthalenes, or a pharmaceutically acceptable salt or prodrug thereof.

9. The use as claimed in any one of the preceding claims, wherein the anti-muscarinic agent is selected from the group consisting of alvameline chloride, bethanechol chloride, darifenacin chloride, dicyclomine hydrochloride, emepronium carrageenate, hyoscyamine sulfate, imipramine hydrochloride, oxybutynin chloride, S-oxybutynin chloride, propantheline bromide, propiverine chloride, revatropate chloride, temiverine chloride, terodiline chloride, tolterodine or a pharmaceutically acceptable salt thereof, trospium chloride, vamicamide chloride, zamifenacin chloride, AH-9700, FK-584, J-104135, KRP-197, YM-905, and YM-46303.

10. The use as claimed in claim 9, wherein the anti-muscarinic agent is selected from the group consisting of oxybutynin chloride, S-oxybutynin chloride, propantheline bromide, propiverine chloride, tolterodine or a pharmaceutically acceptable salt thereof, and trospium chloride.

11. The use as claimed in claim 10, wherein the anti-muscarinic agent is oxybutynin chloride.

12. The use as claimed in claim 10, wherein the anti-muscarinic agent is S-oxybutynin chloride.

13. The use as claimed in claim 10, wherein the anti-muscarinic agent is propantheline bromide.

14. The use as claimed in claim 10, wherein the anti-muscarinic agent is propiverine chloride.

15. The use as claimed in claim 10, wherein the anti-muscarinic agent is tolterodine or a pharmaceutically acceptable salt thereof.

16. The use as claimed in claim 15, wherein the anti-muscarinic agent is tolterodine tartrate.

17. The use as claimed in claim 10, wherein the anti-muscarinic agent is trospium chloride.

18. A pharmaceutical composition comprising
an amount of an anti-muscarinic agent,
an amount of a cyclooxygenase inhibitor or prodrug thereof, and
a pharmaceutically acceptable carrier,
for use in the treatment or prophylaxis of interstitial cystitis.
